# EUROPEAN PATENT APPLICATION

(11) **EP 3 275 432 A1**
(43) Date of publication of application: **31.01.2018**
(21) Application number: 16181058.5
(22) Date of filing: 25.07.2016
(51) Int. Cl.: A61K 9/20, A61K 31/00

(54) **DOSAGE FORM WITH ACE INHIBITOR**

(71) Applicant: H e x a l Aktiengesellschaft, 83607 Holzkirchen (DE)
(72) Inventor: WOLF, Claudia, 83607 Holzkirchen (DE)
(74) Representative: Prüfer & Partner mbB Patentanwälte · Rechtsanwälte

(57) **Abstract**

The present invention is directed to a pharmaceutical dosage form exhibiting improved stabilization of an angiotensin-converting-enzyme inhibitor (ACE inhibitor) present therein. The dosage form comprises the angiotensin-converting-enzyme inhibitor and a stabilizer and has at least two separate portions, wherein the angiotensin-converting-enzyme inhibitor is contained in one of the portions and the stabilizer compound is contained in another portion. The dosage form can make use of an intra- granular/extra-granular structure, wherein preferably the ACE inhibitor compound is placed within the intra-granular portion and the stabilizer is placed in the extra-granular portion, or vice versa the stabilizer is placed within the intra-granular portion and the ACE inhibitor compound is placed in the extra-granular portion, and further alternatively when adopting a core/shell structure, such that the ACE inhibitor is contained in the core and the stabilizer compound is contained in a surrounding layer, or the ACE inhibitor is contained in a surrounding layer and the stabilizer compound is contained in the core, wherein optionally an intermediate layer, such as a layer basically formed from a film-forming polymer, can be provided between the core and the surrounding layer.

## Description

### Field of the invention

The present invention is directed to a pharmaceutical dosage form of an angiotensin-converting-enzyme inhibitor (ACE inhibitor) providing improved stability, and to a process for producing the same.

### Background of the invention

Angiotensin-Converting-Enzyme ("ACE") inhibitors are useful as anti-hypertensives. ACE inhibitors are formulated as tablets, capsules or elixirs for oral administration or in sterile solutions or suspensions for parenteral administration.

ACE inhibitors can be divided into three groups based on their molecular structure, namely dicarboxylate-containing agents (e.g. ramipril, enalapril, quinapril and others), sulfhydryl-containing agents (e.g. captopril, zifenopril) and phosphonate-containing agents (e.g. fosinopril). ACE inhibitors are susceptible to degradation, since in particular ester and/or amide groups present in the respective ACE inhibitor molecule may be subject to degradation reactions, undesirably leading to a reduced amount of the active compound and an increased amount of impurities. This particularly applies to dicarboxylate-type ACE inhibitors. Representatively, the formation of typical impurities is illustrated for ramipril, where major impurities are denoted impurity D and impurity E:

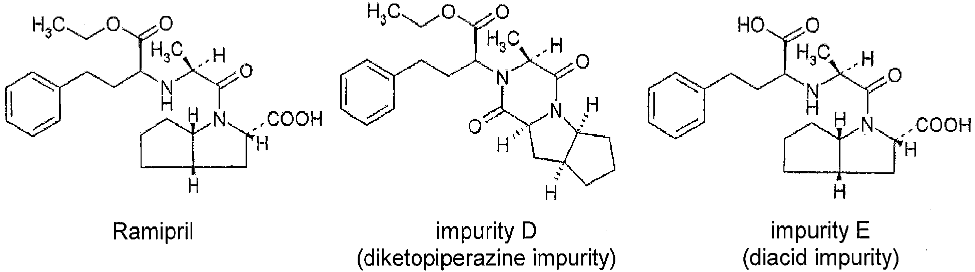

Many attempts have been made to provide stable pharmaceutical formulations and dosage forms, e.g. tablets, of ACE inhibitors.

For example, EP 317 878 A1 refers to the susceptibility of ACE inhibitors to degradation. In order to address the stability issues, the ACE inhibitor is stabilized in the formulation by, prior to the compression of the formulation, applying a protective coating of polymeric film-formers around the ACE inhibitor ingredient, by mixing of the ACE inhibitor with a buffer, or by mixing of the ACE inhibitor with a buffer and additionally providing the protective polymer coating. Applying a protective coating, however, is time-consuming and expensive. The inclusion of a polymer film former in a coating for the ACE inhibitor may also interfere with a desired dissolution profile of the ACE inhibitor compound from the formulation.

US 2003/0215526 A1 describes some pathways of degradation of ACE inhibitors, i.e. the hydrolysis of ester groups and/or oxidation and internal cyclization thereby forming various substituted diketopiperazines. To address the degradation problem an intimate mixture of an ACE inhibitor with alkali or alkaline earth metal carbonate in a greater than stoichiometric amount based on the amount of the ACE inhibitor is proposed, which thus does not involve a coating of the ACE inhibitor. Relatively high amounts of stabilizing salts are required.

Therefore, creating and maintaining stability of a given ACE inhibitor in a pharmaceutical dosage form, in particular in solid form such as a tablet, is a challenging task.

It is an object of the present invention to provide pharmaceutical dosage forms comprising ACE inhibitors with improved stability. There is also a need of providing improved processes for preparing stable pharmaceutical dosage forms of ACE inhibitors.

### Summary of the invention

The present invention provides a pharmaceutical dosage form as defined in claim 1. Preferred embodiments are set forth in the subclaims. The present invention further provides a process for producing a pharmaceutical dosage form according to claim 12, with preferred embodiments being set forth in the subclaims to claim 12. The present invention moreover provides a solid dosage form comprising a dicarboxylate-type ACE inhibitor according to claim 14.

In the present invention, it has been surprisingly found that stabilization is affected and insufficient if a stabilizer is in direct contact with the ACE inhibitor. It has been found that stabilization is substantially enhanced if the stabilizer is present within the pharmaceutical dosage form in a form essentially or completely separated from the ACE inhibitor. Accordingly, the basic concept of claim 1 defines that the dosage form comprises at least two separate portions; the ACE inhibitor is contained in at least one of the portions and the stabilizer compound is contained in at least one portion different from the portion(s) containing the ACE inhibitor. Moreover, the embodiments of claims 2, 3 and 4 provide for efficient separation between the ACE inhibitor and the stabilizer by placing them in different portions of a dosage form, while at the same time effectively utilizing the stabilizing effect of the stabilizer. It has been found that stability of the ACE inhibitor within a common dosage form is particularly enhanced if the dosage form basically makes use of an intra-granular/extra-granular structure where the ACE inhibitor compound is placed within an intra-granular portion and the stabilizer is placed in an extra-granular portion. In further embodiments, the stabilizer can in turn be placed within an intra-granular portion, whereas the ACE inhibitor compound is placed in an extra-granular portion. Alternatively, a core/shell structure can be adopted, such that the ACE inhibitor is contained in the core and the stabilizer compound is contained in a surrounding layer, or the ACE inhibitor is contained in a surrounding layer and the stabilizer compound is contained in the core, wherein optionally at least one intermediate layer, such as a layer formed by using a film-forming polymer, can be provided between the core and the surrounding layer.

Provided that the aforementioned essential or complete separation of the ACE inhibitor with the stabilizer is made, it is possible that the same or another ACE inhibitor may additionally be present in one or more additional portion(s) different from the stabilizer-containing portion. The present invention allows to avoid a homogenous or even an intimate mixture of an ACE inhibitor with a stabilizer, and further allows to preferably avoid a polymer coating of the ACE inhibitor substance itself, where either the ACE inhibitor compound alone or its admixture with a stabilizer is directly coated.

Further, the constitution of the pharmaceutical dosage form according to the present invention allows to achieve a quick release of the ACE inhibitor from the dosage form, which is unaffected by a polymer coating of the ACE inhibitor substance itself, and thus is favorable in terms of bioavailability.

The pharmaceutical dosage form according to the present invention provides excellent stability to the contained ACE inhibitor compound, better than conventional dosage forms in which the stabilizer compound is in close or even intimate contact with the ACE inhibitor. It has been found that less contamination based on reactions between the ACE inhibitor and the stabilizer compound occurs owing to a sufficient separation between the stabilizer compound and the ACE inhibitor by placing them in different portions of the pharmaceutical dosage form, in particular when the stabilizer is contained in the extra-granular portion and not in the intra-granular portion while the ACE inhibitor is contained in the intra-granular portion. Because the stabilizer is not in contact with the ACE inhibitor, the amount of impurities (such as the diketopiperazine impurity and/or the diacid impurities illustrated in case of dicarboxylate-type ACE inhibitors, e.g. the illustrated impurities D and E in case of ramipril) is significantly lower in the dosage forms according to the present invention. In addition, according to the present invention there is no need of a time-consuming and expensive coating procedure. Moreover, without the need for, and thus a detriment effect of, a direct polymer coating on the ACE inhibitor compound, the coating cannot affect the release of the ACE inhibitor from the dosage form. A desired release profile, such as a quick and immediate release, is therefore properly achieved.

The present invention is also directed to a process for producing a pharmaceutical dosage form comprising an angiotensin-converting-enzyme inhibitor or a pharmaceutically acceptable salt thereof and at least one pharmaceutically acceptable excipient or carrier, comprising the following steps:
a) mixing the angiotensin-converting-enzyme inhibitor with the at least one pharmaceutically acceptable excipient or carrier,
b) wet granulating the obtained blend,
c) mixing the granulate obtained in step b) with a stabilizer compound and optionally further pharmaceutically acceptable excipients, and
d) obtaining a resulting pharmaceutical formulation from the mixture of step c); or
a') mixing a stabilizer compound with at least one pharmaceutically acceptable excipient or carrier,
b') wet granulating the obtained blend,
c') mixing the granulate obtained in step b') with the angiotensin-converting-enzyme inhibitor and optionally further pharmaceutically acceptable excipients, and
d') obtaining a resulting pharmaceutical dosage form from the mixture of step c'); or
a") preparing a core comprising a stabilizer compound and at least one pharmaceutically acceptable excipient or carrier,
b") optionally applying an intermediate layer, preferably a layer formed from a film-forming polymer, onto the core obtained in step a"),
c") applying a layer comprising the angiotensin-converting-enzyme inhibitor onto the core obtained in step a"), or onto the intermediate layer optionally obtained in step b"), and
d") obtaining a resulting pharmaceutical dosage form from step c"); or a'") preparing a core comprising the angiotensin-converting-enzyme inhibitor and at least one pharmaceutically acceptable excipient or carrier,
b"') optionally applying an intermediate layer, preferably a layer formed from a film-forming polymer, onto the core obtained in step a'"),
c"') applying a layer comprising a stabilizer compound onto the core obtained in step a"'), or onto the intermediate layer optionally obtained in step b'"), and
d"') obtaining a resulting pharmaceutical dosage form from step c"').

In steps b) and b'), a wet granulation process is carried out with a suitable solvent, such as water, aqueous media or an organic solvent or mixtures thereof. The use of an organic solvent, either as a mixture with water or preferably only the organic solvent without adding water, is preferred. It has been found that if, in accordance with the preferred process embodiment, an organic solvent is used, stickiness of the ACE inhibitor substance is significantly reduced, and a stable pharmaceutical dosage form of an ACE inhibitor is provided which allows for quick release thereof. During or after the wet granulation step, the solvent is suitably removed. For example, fluid bed drying can be used.

Anyone of the above specified steps d), d'), d") and d"') optionally includes tableting, in particular compressing, or filling into capsules. As a further option, a resulting dosage form can be further finally coated. The steps that involve applying a layer, i.e. steps b") and c") or b'") and c'"), can be typically carried out by a conventional film-coating process.

The present invention is also directed to a solid dosage form, such as a tablet, obtained by formulating, e.g. compressing, the above specified portions of the dosage form according to the present invention, and more specifically a solid dosage form, such as a tablet, comprising ramipril or a pharmaceutically acceptable salt thereof.

Within the present invention, the term "angiotensin-converting-enzyme inhibitor" (or shortly "ACE inhibitor") means a compound capable of inhibiting the angiotensin-converting-enzyme (ACE). Whether a compound has the property of ACE inhibition can be determined by a test using commercially available ACE assay kits, or tests known from and described in the literature. In structural terms, the ACE inhibitor typically falls into three groups based on their characteristic molecular structure, namely dicarboxylate-containing agents, sulfhydryl-containing agents and phosphonate-containing agents, and due to common structurally sensitive moieties can be considered common for applying the concept of the present invention. Specifically, the angiotensin-converting-enzyme inhibitor useful in the present invention is prone to degradation.
Further, the meaning of the term "angiotensin-converting-enzyme inhibitor" respectively "ACE inhibitor" includes the basically active ACE inhibitory compound itself, as well as pharmaceutically acceptable salts thereof and derivatives thereof, provided that such salts and derivatives of the corresponding basically active compounds themselves have ACE inhibitory activity.

The terms "intra-granular portion" and "extra-granular portion" as used herein means the respective portions or parts of a pharmaceutical dosage form, wherein the intra-granular portion has been obtained by the formulation of a granulate. Suitable excipients are typically used to formulate the granulate. The granulate can then be subjected to a further step of mixing with constituents of the extra-granular portion. The extra-granular portion is formed for example with appropriate excipients in a compression mass suitable for preparing a tablet; in another example, the extra-granular portion is formed by appropriate excipients to fill a capsule.

The term "stabilizer" as used herein means a compound capable of stabilizing the given ACE inhibitor in the pharmaceutical dosage form of the present invention. Stabilizer and ACE inhibitor are commonly comprised in the pharmaceutical dosage form, such as in the same dosage form, e.g. the same tablet or capsule.

### Detailed description of the invention

In the particularly preferred embodiment, the present invention is directed to a pharmaceutical dosage form comprising an intra-granular portion and an extra-granular portion, wherein the intra-granular portion comprises an angiotensin-converting-enzyme inhibitor or a pharmaceutically acceptable salt thereof and at least one pharmaceutically acceptable excipient or carrier, and wherein the stabilizer compound is present in the extra-granular portion, optionally and preferably together with further pharmaceutically acceptable excipient or carrier (the latter being same or different from those present in the intra-granular portion). Preferably, the stabilizer compound is present only in the extra-granular portion and absent from the intra-granular portion - absence in the sense that the stabilizer compound is not purposively added to the composition of the intra-granular portion. Alternatively, the amount of the stabilizer compound in the intra-granular portion is controlled to be as low as the stability performance is still improved, as can be measured by impurity generation such as total impurities being lower than 0.5wt.%, preferably lower than 0.2wt.%, compared to a situation where the stabilizer and ACE inhibitor compounds were present as a homogeneous mixture. This can be achieved, for instance, by limiting the amount of stabilizer in the intra-granular portion to at most 0.5wt.%, respectively related to the total weight of the final pharmaceutical dosage form.
Furthermore, in the preferred embodiment where the angiotensin-converting-enzyme inhibitor is present in the intra-granular portion, the compound itself is preferably not coated by a polymer.
Analogous features apply to the other embodiments of the present invention with respect to either total absence or limited amount proportions of stabilizer in the same portion of the dosage form as the ACE inhibitor.
In addition to the thus obtained essential or complete separation of the ACE inhibitor and the stabilizer according to the described embodiments, it is possible that the same or another ACE inhibitor may additionally be present in one or more further portion(s), which yet is(are) still different from the stabilizer-containing portion. For instance, it is possible that such additional ACE inhibitor-containing portion is provided by a separate active ingredient-containing layer. For the ease and robustness of design and manufacture, it is possible however to effect the separation in a simplest form by placing the ACE inhibitor only in one of the portions while placing the stabilizer only in a different one of the portions.

The present invention is particularly directed to a pharmaceutical dosage form comprising an intra-granular portion and an extra-granular portion, wherein the intra-granular portion comprises an angiotensin-converting-enzyme inhibitor or a pharmaceutically acceptable salt thereof and at least one pharmaceutically acceptable excipient or carrier, and wherein a stabilizer compound is only comprised in the extra-granular portion.

The ACE inhibitor present in the pharmaceutical dosage form according to the present invention is suitably selected from the groups of dicarboxylate-containing agents, sulfhydryl-containing agents and phosphonate-containing agents, such as ramipril, perindopril, captopril, enalapril, lisinopril, indolapril, quinapril, alacepril, trandolapril, CGS 13928 C (Serradell and Casta, Drugs of the Future 1985,10(3),190), moexipril, perindopril, benazepril, ceranapril, cilazapril, delapril, fosinopril, imidapril, libenzapril, moveltipril, spirapril, and zofenopril. Preferably, the ACE inhibitor is a dicarboxylate-based compound selected from the group consisting of ramipril, perindopril, enalapril, lisinopril, indolapril, quinapril, alacepril, trandolapril, moexipril, perindopril, benazepril, cilazapril, delapril, imidapril, libenzapril, and spirapril, more preferably is ramipril. Included are the pharmaceutically acceptable salts and derivatives of the mentioned ACE inhibitor molecules, having ACE inhibition property.

The stabilizer compound according to the present invention stabilizes the ACE inhibitor against degradation. More specifically the stabilizer compound protects the ACE inhibitor from impurity generation. Owing to its efficient stabilization effect, the stabilizer is preferably an alkaline substance (typically a base) or a buffer substance, in particular an alkaline substance or a buffer substance capable of imparting, to the composition comprising the ACE inhibitor and the stabilizer upon its dissolution or dispersion in water, a pH value in water of pH 4.5-7.0, preferably a pH of 5.0-6.0 and particularly around a pH of 5.5 (e.g. ±0.25). The pH value is suitably measured at room temperature (e.g. 21-23°C) after dissolving or dispersing the dosage form comprising the ACE inhibitor and the stabilizer in water in an amount sufficient for dissolving or dispersing it; practically, a single dosage form such as a single tablet may be dissolved or dispersed in about 10 to 20 ml water, e.g. 15ml, and the pH is measured.

The stabilizer compound suitably comprises, preferably is, an alkalizing agent, for example selected from the group consisting of polyhydric alkanolamines such as tris(hydroxymethyl)-aminomethane (2-amino-2-hydroxymethyl-propane-1,3-diol), triethanolamine, diethanolamine and N-methyl-D-glucamine (meglumine), sodium dihydrogen phosphate, trisodium citrate, sodium carbonate, sodium hydrogen carbonate, magnesium oxide, magnesium carbonate, calcium carbonate, calcium hydrogen carbonate, sodium citrate, and potassium citrate. The stabilizer preferably contains, more preferably is, at least one of the mentioned polyhydric alkanolamines; in particular the stabilizer is tris(hydroxymethyl)aminomethane (2-amino-2-hydroxymethyl-propane-1,3-diol, sometimes also abbreviated as THAM or Tris).

The dosage form can contain the ACE inhibitor at a desired and suitable strength, which may also differ depending on the specific ACE inhibitor compound used. Different dosages of a given ACE inhibitor are possible as well, allowing varying dosage regimen using a starting dose followed by another dose. A dosage may be adjusted according to a clinical response.

For example, respective ACE inhibitor compounds with respective suitable dosages include ramipril 2.5 mg to 20 mg (e.g. 10 mg); benazepril 10 mg to 80 mg; captopril 50 mg to 450 mg; enalapril 5 mg to 40 mg; fosinopril 10 mg to 80 mg; lisinopril 10 mg to 80 mg; moexipril 7.5 mg to 30 mg; perindopril 4 mg to 16 mg; quinapril 10 mg to 80 mg; trandolapril 2 mg to 8 mg; and others.

The total amount of the stabilizer compound is chosen to provide a sufficient stabilization effect, for instance is in a range from about 0.1 to about 10 wt%, preferably about 0.3 to about 5 wt%, more preferably about 1.0 to about 1.5wt%, respectively in relation to the total amount of the dosage form.

According to the present invention preferably no stabilizer compound is added to and accordingly no or essentially no stabilizer compound is present in the portion of the pharmaceutical dosage form of the present invention where the ACE inhibitor is present.

It is further preferred that no ACE inhibitor is added to and accordingly no ACE inhibitor is present in the portion of the pharmaceutical dosage form of the present invention where the stabilizer is present.

The type and amount of the excipient or carrier can be chosen and adjusted depending on the portion it is to be included in, that is in the portion together with the ACE inhibitor and/or in the portion together with the stabilizer.

Within the portion of the pharmaceutical dosage form where the ACE inhibitor is present, at least one pharmaceutically acceptable excipient or carrier can be contained in a suitable amount, for example at an amount of at least 3 wt%, preferably at least 5 wt%, and possibly up to 70wt% or 80wt% or 90wt%, relative to the weight of the angiotensin-converting-enzyme inhibitor in the corresponding same portion, such as the same intra-granular portion, the same extragranular portion, the same layer or the same core.

The at least one pharmaceutically acceptable excipient or carrier suitable for being incorporated into the intra-granular portion is preferably selected from the group consisting of fillers and binders and mixtures thereof.

The filler is for example selected from the group consisting of microcrystalline cellulose, lactose (in particular lactose monohydrate and/or anhydrous lactose), sucrose, dextrose, mannitol, sorbitol, starch, including various starch types and derivatives such as pregelatinized starch, cellulose, dibasic calcium phosphate (dehydrated or anhydrous), calcium sulphate, etc. The binder is for example selected from the group consisting of cellulose derivatives, microcrystalline cellulose, gelatine, polymethacrylate, polyvinylpyrrolidone, starch, including various starch types and derivatives, preferably is selected from cellulose derivatives, such as hydroxypropyl cellulose, methyl cellulose, carboxymethyl cellulose, hydroxypropylmethyl cellulose, hydroxypropyl cellulose and hydroxyethyl cellulose.

The at least one pharmaceutically acceptable excipient or carrier suitable for being incorporated into the extra-granular portion may include, without being limited to, disintegrants, fillers, binders, lubricants, glidants, colorants, and flavouring agents, and mixtures thereof. Possible fillers and binders may include the same or other types and/or amounts as those described for the intra-granular portion. Preferred lubricants are sodium stearyl fumarate and magnesium stearate, and preferred disintegrants are croscarmellose sodium, sodium starch glycolate, crospovidone and low-substituted hydroxypropyl cellulose (L-HPC). When fillers and/or binders are used for the extra-granular portion, it is further preferred that a part of the fillers or binders or mixtures thereof is present in the intra-granular portion, and a remainder part of this excipient is present in the extra-granular portion.

The present invention is also directed to a dosage form obtained by formulating the respective portions described according to the present invention. Formulation methods include, for example, compression, core and layer formation, and the like.

When a tablet is formulated according to the present invention, for example dry granulation and wet granulation can be used. The dry or wet mass can subsequently be compressed. Wet granulation is preferred. Another suitable method includes preparing a core and applying one or more layers onto the core, e.g. by way of film-coating.

The present invention is also directed to the above specified process for producing the pharmaceutical dosage form comprising the angiotensin-converting-enzyme inhibitor, the stabilizer and the at least one pharmaceutically acceptable excipient or carrier. A preferred embodiment of the process according to the present invention comprises the steps of:
a) mixing the angiotensin-converting-enzyme inhibitor with the at least one pharmaceutically acceptable excipient or carrier,
b) wet granulating the obtained blend, using an aqueous medium or, preferably, an organic solvent, which typically includes or is followed by removing the solvent from the granulated blend, and
c) mixing the granulate obtained in step b) with a stabilizer compound and optionally further pharmaceutically acceptable excipients, and
d) obtaining a resulting pharmaceutical dosage form from the mixture of step c).

With respect to the process according to the present invention, it is noted that ACE inhibitors, such as ramipril, tend to stick to parts of formulation equipment, such as punches of tabletting machines. This effect increases with the concentration of the ACE inhibitor in the dosage form. For reasons of patient compliance, however, it is not desirable to produce large dosage forms, e.g. tablets, with low concentration of an ACE inhibitor. Therefore, handling the ACE inhibitors in the dry state, e.g. dry granulation or direct tableting of powder mixtures, has been found to be difficult. However, it was found that the sticking problem is overcome by the use of a granulation liquid, in particular when the granulation liquid comprises, preferably consists of, an organic solvent. Besides significantly facilitating the wet granulation by the use of organic medium, avoidance of water further reduces the risk of instability and decomposition of the ACE inhibitor during the formulation process.

The organic solvent is suitably selected from the group of Q3C class 1 solvents, preferably is selected from the group consisting of isopropanol, ethanol, acetone and mixtures thereof, more preferably is isopropanol.

The separation concept according to the present invention not only generally reduces conventionally known ACE-specific impurities such as diacid and diketopiperazine type impurities. In addition it was found that the separation of a specific class compound, namely a carboxylate-type ACE inhibitor, from a specific and preferred type of stabilizer, which can be characterized as polyhydric alkanolamine type stabilizer, avoids an impurity which would be formed by a reaction between this carboxylate-type ACE inhibitor compound and the polyhydric alkanolamine compound. Such impurity can be defined by the general formula X-O-Y, wherein X denotes the polyhydric alkanolamine moiety, O represents one or more of the OH-groups of the polyhydric alkanolamine, and Y denotes the carboxylate-type ACE inhibitor derived structural moiety. Typically, the formed impurity can be characterized as an esterified or transesterified reaction product with respectively the carboxylate group or the methoxy ester group of the ACE inhibitor compound. This can be exemplified in the following by the reaction product of tris(hydroxymethyl)-aminomethane (Tris) with ramipril (denoted ramipril-Tris-ester):

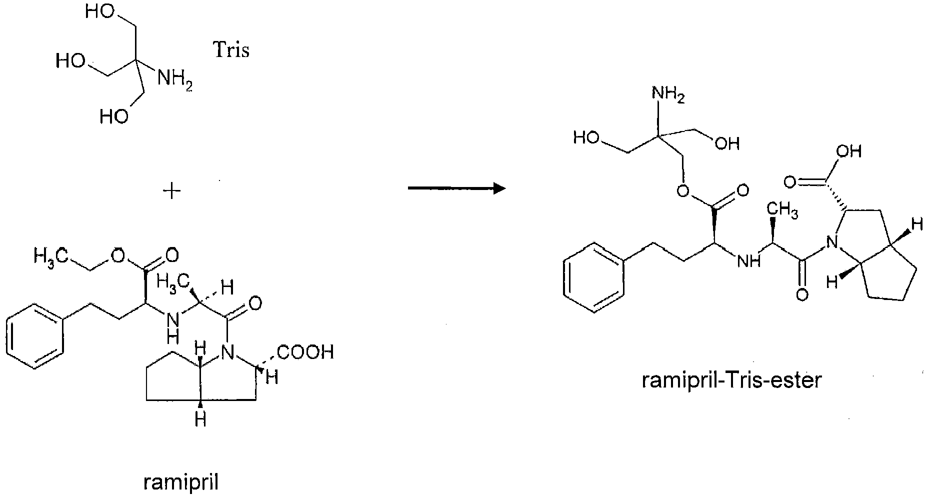

As a result of finding and identifying the potentially generated new impurity, which is formed when the carboxylate-type ACE inhibitor compound would be homogeneously combined/mixed with the polyhydric alkanolamine type stabilizer, the present invention makes it possible to provide a solid dosage form comprising the carboxylate-type ACE inhibitor and as a stabilizer the polyhydric alkanolamine, wherein the amount of impurity formed by a reaction between the carboxylate-type ACE inhibitor compound and the polyhydric alkanolamine compound is at most 0.20 wt.-%, preferably at most 0.10 wt.-%, and more preferably at most 0.05 wt.-%, relative to the total mass of the solid dosage form and measurable after a suitable time period under suitable test conditions, such as after one month storage at 40°C/75% relative humidity in an aluminium blister package.

Accordingly, when the specific polyhydric alkanolamine is chosen as the stabilizer, however since the contact between the carboxylate-type ACE inhibitor compound and the specific stabilizer compound is reduced to a minimum or is entirely avoided, the reaction-derived impurity is controlled to not exceed the specified limits, which otherwise did exceed said limits under the same storage conditions in a comparison solid dosage form in which there is contact between both substances, in particular if homogenously mixed.

In this particular aspect of the present invention, the carboxylate-type ACE inhibitor compound may be selected, as described above, from ramipril, perindopril, enalapril, lisinopril, indolapril, quinapril, alacepril, trandolapril, moexipril, perindopril, benazepril, cilazapril, delapril, imidapril, libenzapril, and spirapril, more preferably is ramipril. In a particularly selected embodiment the inventive concept is applied to, and hence the ACE inhibitor is selected to be, ramipril or a pharmaceutically acceptable salt thereof. In this embodiment, the solid dosage form is preferably a tablet, optionally coated.

As further described above, the polyhydric alkanolamine may preferably be selected from the group consisting of tris(hydroxymethyl)-aminomethane (2-amino-2-hydroxymethyl-propane-1,3-diol; Tris), triethanolamine, diethanolamine and N-methyl-D-glucamine (meglumine), in particular is tris(hydroxymethyl)-aminomethane.

### Examples

### Example 1 and Comparative Example 1

Without being limited thereto, the present invention is illustrated by the use of ramipril as a typical and representative ACE inhibitor which is prone to degradation.

Tablets containing 10 mg of ramipril and the following ingredients listed in Table 1 (mass indications in mg per tablet) were prepared. Isopropanol was used for wet granulation.

**Table 1:**

| Ingredient | Example 1 | Comparative Example 1 |
|---|---|---|
| Ramipril | 10.00 | 10.00 |
| microcrystalline cellulose | ad 100.00 | ad 100.00 |
| 2-amino-2-hydroxymethyl-propane-1,3-diol (Tris) | 1.25 | 1.25 |
| hyprolose | 2.00 | 2.00 |
| sodium stearyl fumarate | 1.00 | 1.00 |
| croscarmellose sodium | 2.50 | 2.50 |
| Isopropanol* | q.s. | q.s. |

| | | |
|---|---|---|
| * not contained in the product | | |

The 10mg amount of ramipril was mixed with 30mg of the microcrystalline cellulose and with 1 mg hyprolose (hydroxypropyl cellulose). In Comparative Example 1 the 2-amino-2-hydroxymethyl-propane-1,3-diol (Tris) was already added to the blend before granulation thereof. The respective blend was granulated in a suitable granulator (e.g. a vertical type granulator) with a solution of hyprolose (the remaining 1 mg) in isopropanol. The wet mass was dried to remove the solvent.

After the granulate was thus obtained, the remainder of the microcrystalline cellulose and sodium stearyl fumarate and croscarmellose sodium were mixed with the granulate. Different from Comparative Example 1, in Example 1 Tris was mixed with the previously prepared granulate to become part of the extra-granular portion. The obtained mixture was compressed to tablets with a weight of 100 mg. The tablets were packaged in aluminium blisters.

As a control, another tablet was prepared without any stabilizer (Tris).

Stability tests of the packaged tablets were conducted at 40 °C and 75% relative humidity. After one month impurities/contamination were determined by HPLC analysis. The results are shown in Table 2.

**Table 2:**

| /wt% | Example 1 | Comparative Example 1 |
|---|---|---|
| Impurity D | 0.76 | 2.25 |
| Impurity E | 0.08 | 0.20 |
| Contamination based on reaction of ramipril with Tris (ramipril-Tris-ester) | 0.05 | 0.28 |

A tablet prepared without stabilizer (Tris) contained a higher amount of impurities than Comparative Example 1.

The comparison shows that the pharmaceutical dosage form according to the present invention contains significantly less impurity D and impurity E, which impurities shall be avoided by the stabilizer compound 2-amino-2-hydroxymethyl-propane-1,3-diol (Tris). Moreover, also substantially less contamination based on reaction of ramipril with Tris (ramipril-Tris-ester) is contained in the dosage form according to the invention, since the contact between both compounds is reduced by providing stabilizer only in the portion(s) where the ACE inhibitor is not present, in the present example in the extra-granular portion.

### Examples 2 to 11

According to further Examples 2 to 11 tablets are prepared in the same way as in Example 1, however using the intra-granular ingredients for preparing granules by wet granulation and the extra-granular ingredients for the subsequent compression mass of preparing respective tablets as set forth in the following Tables 3 and 4 (mass indications in mg per tablet, noting that the wet granulation solvent is removed during the process and is not present in the final tablet; the term "GRA" means granulation, and the term "FB" means fluid bed):

**Table 3**

| | **Example 2** | **Example 3** | **Example 4** | **Example 5** | **Example 6** |
|---|---|---|---|---|---|
| **Ingredients Granules:** | | | | | |
| Ramipril | 10.00 | 10.00 | 10.00 | 10.00 | 10.00 |
| MCC | 3.00 | 3.00 | | 15.50 | 15.50 |
| Mannitol | | | 15.50 | | |
| HPC | 2.00 | 2.00 | | 2.00 | 2.00 |
| HMPC | | | 2.00 | | |
| Isopropanol | 3.00 | 3.00 | | 3.67 | 3.00 |
| Water | | | 3.00 | | |
| type of wet granulation | Organic GRA | Organic GRA | Aqueous GRA | Organic GRA | Organic GRA |

| Ingredients **Compression Mass:** | | | | | |
|---|---|---|---|---|---|
| MCC | 74.25 | 79.75 | | 67.25 | |
| Prosolv 90 LM | | | | | 68.50 |
| Mannitol | | | 67.25 | | |
| Sodiumstearylfumarate | 0.25 | 0.25 | 0.25 | 0.25 | 0.25 |
| Croscarmellose sodium | 2.50 | 2.50 | 2.50 | 2.50 | 2.50 |
| Na-hydrogencarbonate | 8.00 | | | | |
| TRIS | | 2.50 | 2.50 | | 1.25 |
| Magnesium oxide | | | | 2.50 | |
| Sum: | 100.00 | 100.00 | 100.00 | 100.00 | 100.00 |

**Table 4**

| | **Example 7** | **Example 8** | **Example 9** | **Example 10** | **Example 11** |
|---|---|---|---|---|---|
| **Ingredients Granules:** | | | | | |
| Ramipril | 10.00 | 10.00 | 10.00 | 10 | 10 |
| MCC | 15.50 | | | 35.6 | 35.6 |
| Mannitol | | 15.50 | 15.50 | | |
| HPC | 2.00 | 2.00 | 2.00 | | |
| HMPC | | | | 2.4 | 2.4 |
| Isopropanol | 3.00 | 3.00 | 3.00 | | |
| Water | | | | 66.44 | 66.44 |
| type of wet granulation | Organic GRA | Organic GRA | Organic GRA | Aqueous FB | Aqueous FB |

| **Ingredients Compression Mass:** | | | | | |
|---|---|---|---|---|---|
| MCC | | | | 47.18 | 40.43 |
| Prosolv 90 LM | | | 68.50 | | |
| Blue color | | | | 0.07 | 0.07 |
| Sodiumstearylfumarate | 0.25 | 0.25 | 0.25 | 1 | 1 |
| MCC low moisture | 68.50 | 68.50 | | | |
| Croscarmellose sodium | 2.50 | 2.50 | 2.50 | 2.50 | 2.50 |
| Na-hydrogencarbonate | | | | | 8 |
| TRIS | 1.25 | 1.25 | 1.25 | 1.25 | |
| Sum: | 100.00 | 100.00 | 100.00 | 100.00 | 100.00 |

## Claims

1. A pharmaceutical dosage form comprising an angiotensin-converting-enzyme inhibitor and a stabilizer, wherein the dosage form comprises at least two separate portions, **characterized in that** the angiotensin-converting-enzyme inhibitor is contained in at least one of the portions and that the stabilizer compound is contained in another portion.

2. The pharmaceutical dosage form according to claim 1, wherein said at least two separate portions comprise an intra-granular portion and an extra-granular portion, wherein the angiotensin-converting-enzyme inhibitor is contained in the intra-granular portion and the stabilizer compound is contained in the extra-granular portion.

3. The pharmaceutical dosage form according to claim 1, wherein said at least two separate portions comprise an intra-granular portion and an extra-granular portion, wherein the stabilizer compound is contained in the intra-granular portion and the angiotensin-converting-enzyme inhibitor is contained in the extra-granular portion.

4. The pharmaceutical dosage form according to claim 1, wherein said at least two separate portions comprise a core and a layer surrounding the core, wherein the angiotensin-converting-enzyme inhibitor is contained in the core and the stabilizer compound is contained in the surrounding layer, or wherein the angiotensin-converting-enzyme inhibitor is contained in the surrounding layer and the stabilizer compound is contained in the core, wherein optionally at least one intermediate layer is provided between the core and the surrounding layer.

5. The pharmaceutical dosage form according to anyone of claims 1 to 4, wherein the angiotensin-converting-enzyme inhibitor is selected from the group consisting of ramipril, perindopril, captopril, enalapril, lisinopril, indolapril, quinapril, alacepril, trandolapril, CGS 13928 C, moexipril, perindopril, benazepril, captopril, ceranapril, cilazapril, delapril, fosinopril, imidapril, libenzapril, moveltipril, spirapril, and zofenopril, preferably is a dicarboxylate-based compound selected from the group consisting of ramipril, perindopril, enalapril, lisinopril, indolapril, quinapril, alacepril, trandolapril, moexipril, perindopril, benazepril, cilazapril, delapril, imidapril, libenzapril, and spirapril, in particular is ramipril.

6. The pharmaceutical dosage form according to anyone of the preceding claims, wherein the stabilizer compound is an alkaline substance or a buffer substance, preferably an alkaline substance or a buffer substance capable of imparting to the dosage form comprising the angiotensin-converting enzyme inhibitor and the stabilizer a pH value in water of 4.5-7.0, preferably a pH of 5.0-6.0, wherein the pH value is measured at room temperature after dissolving or dispersing the dosage form comprising the angiotensin-converting enzyme inhibitor and the stabilizer in water.

7. The pharmaceutical dosage form according to any one of the preceding claims, wherein the stabilizer compound is an alkalizing agent, preferably selected from the group consisting of polyhydric alkanolamines, sodium dihydrogen phosphate, trisodium citrate, sodium carbonate, sodium hydrogen carbonate, magnesium oxide, magnesium carbonate, calcium carbonate, calcium hydrogen carbonate, sodium citrate, and potassium citrate, more preferably is a polyhydric alkanolamine selected from tris(hydroxymethyl)-aminomethane, triethanolamine, diethanolamine and N-methyl-D-glucamine (meglumine), in particular is tris(hydroxymethyl)-aminomethane.

8. The pharmaceutical dosage form according to any one of the preceding claims, wherein the total amount of the stabilizer compound is from about 0.1 to about 10 wt%, preferably about 0.3 to about 5 wt%.

9. The pharmaceutical dosage form according to any one of the preceding claims, wherein the portion comprising the angiotensin-converting-enzyme inhibitor had been formulated with at least one pharmaceutically acceptable excipient or carrier selected from the group consisting of fillers, binders and mixtures thereof, wherein the filler is preferably selected from the group consisting of microcrystalline cellulose, lactose, mannitol, sorbitol, starch and starch derivatives, cellulose and dibasic calcium phosphate and more preferably is microcrystalline cellulose, and/or wherein the binder is preferably selected from the group consisting of cellulose derivatives, microcrystalline cellulose, polyvinylpyrrolidone, starch and starch derivatives, more preferably is selected from hydroxypropyl cellulose, methyl cellulose, carboxymethyl cellulose, hydroxypropylmethyl cellulose, hydroxypropyl cellulose and hydroxyethyl cellulose.

10. The pharmaceutical dosage form according to claim 2 or 3, wherein the extra-granular portion comprises at least one pharmaceutically acceptable excipient or carrier selected from the group consisting of disintegrants, lubricants, glidants, and, optionally further types and/or amounts of, fillers and binders, and mixtures thereof, preferably wherein the at least one pharmaceutically acceptable excipient or carrier present in the extra-granular portion is selected from the group consisting of microcrystalline cellulose, hydroxypropyl cellulose, croscarmellose sodium, stearate salts, and mixtures thereof.

11. The pharmaceutical dosage form according to any one of claims 1 to 10, which is in the form of a tablet, optionally coated.

12. A process for producing a pharmaceutical dosage form comprising an angiotensin-converting-enzyme inhibitor or a pharmaceutically acceptable salt thereof and at least one pharmaceutically acceptable excipient or carrier, comprising the following steps:
a) mixing the angiotensin-converting-enzyme inhibitor with the at least one pharmaceutically acceptable excipient or carrier,
b) wet granulating the obtained blend, preferably with an organic solvent,
c) mixing the granulate obtained in step b) with a stabilizer compound and optionally further pharmaceutically acceptable excipients, and
d) obtaining a resulting pharmaceutical dosage form from the mixture of step c); or
a') mixing a stabilizer compound with at least one pharmaceutically acceptable excipient or carrier,
b') wet granulating the obtained blend, preferably with an organic solvent,
c') mixing the granulate obtained in step b') with the angiotensin-converting-enzyme inhibitor and optionally further pharmaceutically acceptable excipients, and
d') obtaining a resulting pharmaceutical dosage form from the mixture of step c'); or
a") preparing a core comprising a stabilizer compound and at least one pharmaceutically acceptable excipient or carrier,
b") optionally applying an intermediate layer, preferably a layer formed from a film-forming polymer, onto the core obtained in step a"),
c") applying a layer comprising the angiotensin-converting-enzyme inhibitor onto the core obtained in step a"), or onto the intermediate layer optionally obtained in step b"), and
d") obtaining a resulting pharmaceutical dosage form from step c"); or
a"') preparing a core comprising the angiotensin-converting-enzyme inhibitor and at least one pharmaceutically acceptable excipient or carrier,
b"') optionally applying an intermediate layer, preferably a layer formed from a film-forming polymer, onto the core obtained in step a"'),
C'") applying a layer comprising a stabilizer compound onto the core obtained in step a"'), or onto the intermediate layer optionally obtained in step b"'), and
d"') obtaining a resulting pharmaceutical dosage form from step c"').

13. The process according to claim 12, wherein the produced pharmaceutical dosage form is as defined in any one of claims 1 to 11.

14. A solid dosage form comprising a carboxylate-type ACE inhibitor compound and as a stabilizer a polyhydric alkanolamine compound, wherein the amount of an impurity compound formed by a reaction between the carboxylate-type ACE inhibitor compound and the polyhydric alkanolamine compound is at most 0.20 wt.-%, relative to the total mass of the solid dosage form, measured after one month storage at 40°C/75% relative humidity in an aluminium blister package.

15. The solid dosage form according to claim 14, preferably a tablet, wherein the carboxylate-type ACE inhibitor compound is ramipril, and/or wherein the polyhydric alkanolamine compound is 2-amino-2-hydroxymethyl-propane-1,3-diol.
